# EUROPEAN PATENT SPECIFICATION

(11) **EP 4 427 709 B1**
(45) Date of publication and mention of the grant of the patent: **04.06.2025**
(21) Application number: 24162058.2
(22) Date of filing: 07.03.2024
(51) Int. Cl.: A61F 2/58, A61F 2/78, A61F 5/01, A61F 2/50, A61F 2/54, A61F 2/76

(54) **CUFF AND ORTHOTIC SYSTEM**
MANSCHETTE UND ORTHESENSYSTEM
BRASSARD ET SYSTÈME ORTHÉTIQUE

(30) Priority: 07.03.2023 EP 23160455
(43) Date of publication of application: 11.09.2024
(73) Proprietor: macu4 AG, 8001 Zürich (CH)
(72) Inventor: SCHILLER, Lukas, 8006 Zürich (CH); CHEVROT, Alec, 1002 Chavannes-près-Renens (CH)
(74) Representative: Schulz Junghans Patentanwälte PartGmbB

(56) References cited:
- JP-B2- 6 526 691
- US-A1- 2019 091 043
- US-B1- 9 956 093
- US-B2- 8 062 241
- US-B2- 9 757 266

## Description

The present invention relates to a cuff as well as to an orthotic system. US 9 757 266 B2 discloses the features of the preamble of claim 1.

The orthotic system comprises accessories to serve besides others general assistance of the griping or replacing the griping. The orthotic system is also called an ortho-prosthetic system. In the case of a congenital deformity or amputation, it may be difficult to perform bimanual activities such as cycling. Similarly, holding a shopping bag can become a challenge. Daily life for these people is full of little challenges that an ordinary person would never suspect, let alone imagine. People with a birth defect learn to live with it, but outside help is welcome to ease the effort and mental burden. For people who have suffered an amputation as a result of an accident, for example, it's necessary to put a relearning of skills in place. Tools can facilitate not only their daily life but also their rehabilitation. Similarly, in the event of an accident, surgery or injury, hands may become weakened, sensitive, immobilized for a limited period. Therefore, a rapid provision of support is essential to enable affected individuals to continue their activities and daily routines.

An orthotic system is designed for individuals with partial or complete loss of hand function. This loss may be due to temporary or permanent weakening, hand injury, paralysis, neuromuscular disease, partial absence of the hand or absence due to wrist disarticulation.

An orthotic system designed for forearms is intended to assist individuals with a disability below the wrist. This encompasses various scenarios, including those with only a portion of the palm remaining, small residual fingers, a limited number of fingers, or limited and painful movement capacity. In consequence, the system should be engineered to transmit the force generated by the accessory directly to the forearm.

Existing solutions are often poorly adapted, expensive and restricting in terms of the activities they support.

The problem to be solved by the present invention is to provide a cuff and an orthotic system that ensure comfort through a lightweight, intuitive and modular design, while transferring load forces that occur in the area of the accessories directly onto a limb.

This problem is solved by the subject-matter of the independent claim 1.

Embodiments of the invention are claimed in dependent claims 2 to 12.

A first aspect of the invention relates to a cuff, which is combinable with accessories to form an orthotic system, comprising a cuff base in form of a lower shell and a fixation element, which can be combined or is combined with the lower shell into a sleeve attachable to a limb through direct or indirect interlocking with each other, wherein the lower shell and the fixation element provide a system that can be tightened around the limb by reducing a circumference formed by the lower shell and the fixation element, such that load transfer from an accessory to the limb is enabled.

The cuff can be designed to be positioned on an arm, for instance on a forearm or on an upper arm.

The interlocking between the lower shell and the fixation element comprises a magnetic connection, wherein in a body of the lower shell at least one first magnetic part is provided and in or at the fixation element at least one second magnetic part is provided such that by means of an attracting magnetic force between both magnetic parts the fixation element can be moved onto one side of the body of the lower shell or is or can be held on one side of the body of the lower shell.

The first magnetic part and the second magnetic part do not necessarily both have to be made of a magnetic material, it is sufficient that at least one of the two magnetic parts is made of or comprises a magnetic material, and the other magnetic part can be either made of, or comprise, a magnetisable or magnetic material.

The lower shell comprises at least one first form fit means and the fixation element comprises at least one second form fit means, wherein both form fit means are adapted to realize a form fit connection.

The form fit connection provides an undercut in order to prevent its release of the form fit connection when subjected to a force that is essentially perpendicular to the insertion direction of the form fit connection.

The magnetic parts pull the lower shell and/ or the fixation element into positions in which the form fit connection is established.

Alternatively, the magnetic parts pull the lower shell and/ or the fixation element into positions still spaced apart from each other, so that no form fit connection is established merely by means of the magnetic force.

This facilitates an easy decoupling of the form fit connection. When there is tension in form fit means, the form fit means are pulled into the positions where the form fit connection happens. In one embodiment of the cuff, the fixation element is an upper shell.

Each shell is moulded as an ergonomically adapted cavity, which aligns with the natural shape of the human limb.

The advantage of the fixation element in form of an upper shell is that it significantly increases stability.

Particularly, in this embodiment the cuff comprises at least two shells that can be combined to form one all-around coverage of the limb.

The invention is not limited to the upper and lower positions of the upper shell and the lower shell, but these shells can also be located at other positions on the limb.

The cuff may provide at least one interlocking member in form of at least one lacing element, which is mechanically connected to both shells when the interlocking member is interlocked, wherein the lacing element is adapted in such a way that when shortening an effective length of the lacing element, the distance between the two shells is reduced.

The lacing element may comprise one of the second magnetic parts and/ or one of the second form fit means, in order to provide the magnetic connection and/ or the form fit connection. By manipulating the lacing system, the lacing element is shortened, thereby generating a pulling force within the lacing element, which is transmitted to the second form fit means, which is in turn transferred from the second form fit means to the first form fit means and subsequently into the cuff base or lower shell in order to pull the cuff base or lower shell towards the cuff top or upper shell.

A ratchet fixation system could also be considered as alternative to the lacing system.

In another embodiment of the cuff, the fixation element is a strap system.

The strap system corresponds to the interlocking member. A strap of the strap system can be directly mounted through a slit on the cuff base.

The advantage of a strap system is its high breathability.

It is not excluded from the scope of the present invention that the strap system and the upper shell are used simultaneously.

The strap system may comprise at least one of the second magnetic parts and/ or one of the second form fit means.

In an embodiment, where the fixation element is an upper shell, and/ or in an embodiment in which the fixation element is a strap system, the first form fit means can be at least one geometric interlocking piece, for instance in the form of a hook, which serves as a connection between the two shells or serves as a connection between the lower shell and the strap system, wherein the geometric interlocking piece may be incorporated into the lower shell.

Aforementioned first form fit means or geometric interlocking piece may exist as a separate body, either distinct from both shells or solely from the lower shell and the strap system, respectively.

Aforementioned first form fit means or geometric interlocking piece may be connectable geometrically on one side with the lower shell and linked to the upper shell by the lacing element and its second form fit means.

Aforementioned form fit means or geometric interlocking piece may be attracted to its fitting and held in place by magnetic force generated by the magnetic parts.

At least one strap of the strap system may comprise a hook-and-loop fastener.

The use of such a hook-and-loop fastener simplifies the attachment and the removal of the cuff for the user.

The cuff can be intended for persons with partial or complete loss of the hand function due to temporary or permanent weakening, hand injury, paralysis, neuromuscular disease, partial absence of the hand or absence due to wrist disarticulation.

Through the utilization of the cuff with an appropriate accessory, a lack of gripping dexterity, capability or force can be reacquired or supported. It aims to support the activities of the user such as sports, leisure and daily routines.

The cuff corresponds to the part which comes in direct contact with the limb, in particular the forearm, of the user.

Thereby, the cuff can be operated with one hand, its design is user friendly and ergonomic.

Alternatively, at least one strap of the strap system may comprise at least an elastic strip that can be tensioned and adapted in shape to the shape and size of the limb.

A further aspect of the present invention is an orthotic system, comprising a described cuff and a module mechanically connected or connectable to the cuff, wherein the cuff comprises a first part of a ratchet system and the module comprises a second part of the ratchet system, wherein the first part and the second part have form elements, respectively, which are complementary in form and size.

One of the elements of the cuff and module may comprise a retainer, equipped with one of the first part or second part of the ratchet system and the respective other element of the cuff and module may comprise an insertion element that is intended to be positioned in the retainer, wherein the insertion element is equipped with the respective other part of the first part or second part of the ratchet system and is designed to be pushed into the retainer along a slide-in direction, and due to an application of force to bring the first parts and second parts in a positive locking.

The application of force may be realized by an elastic behaviour of the insertion element that is pushing the respective first part or second part of the ratchet system in the direction of the respective first part or second part at the retainer in order to establish a positive locking between the first parts and the second parts.

The insertion element may be bent while inserting the insertion element into the retainer. Thereby the retainer is configured to absorb the induced bending moment, enabling the application of force needed to establish a positive locking.

The elasticity of the insertion element can be adapted in such a way that the insertion element can be manually pushed against its elastic force and thus be moved by applying a pressing force of at least 3 N, thereby disengaging the first part and second part of the ratchet system from the positive locking. This allows the user to manually remove the positive locking if desired.

The push direction is essentially perpendicular to the slide-in direction.

A plurality of first parts and second parts may be arranged in rows, respectively, so that the positive locking can be established at different positions along the slide-in direction.

At least one of the elements of the orthotic system may be manufactured by additive manufacturing and, more specifically, selective laser sintering. The material can be either PA11 or PA12. Depending on the volume of this product line, another manufacturing technique, such as injection moulding, could be considered, too, as well as other materials.

The prosthetic system may be combined with various accessories, enabling the user to pursue activities such as riding a bike, holding a bag, pulling a suitcase, walking with crutches, kayak or ski.

The invention is further illustrated by the following examples and figures, from which further embodiments and advantages can be drawn. These examples are meant to illustrate the invention but not to limit its scope.

It is shown in
Fig. 1: a first embodiment of the cuff in a perspective view,
Fig. 2: a partial sectional view of the cuff shown in fig. 1 before form fit connection,
Fig. 3: the partial sectional view of the cuff shown in fig. 1 in form fit connection,
Fig. 4: a further embodiment of the cuff shown in fig. 2 in form fit connection,
Fig. 5: a second embodiment of the cuff in a perspective view,
Fig. 6: the underside of the cuff shown in fig. 1,
Fig. 7: an orthotic system in a perspective view,
Fig. 8: a part of the lower shell as well as a part of the insertion element in a first state,
Fig. 9: the part of the lower shell as well as the part of the insertion element in a second state,
Fig. 10: the part of the lower shell as well as the part of the insertion element in a third state, and
Fig. 11: the part of the lower shell as well as the part of the insertion element in a fourth state.

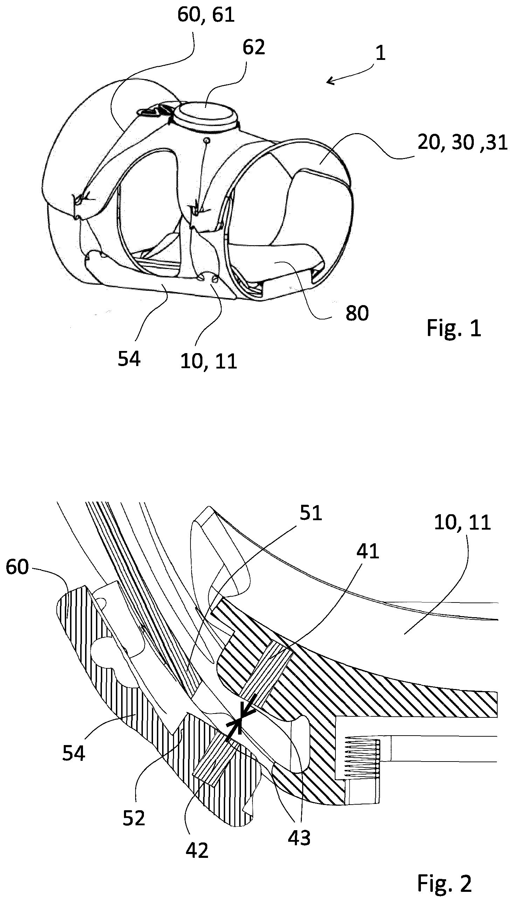

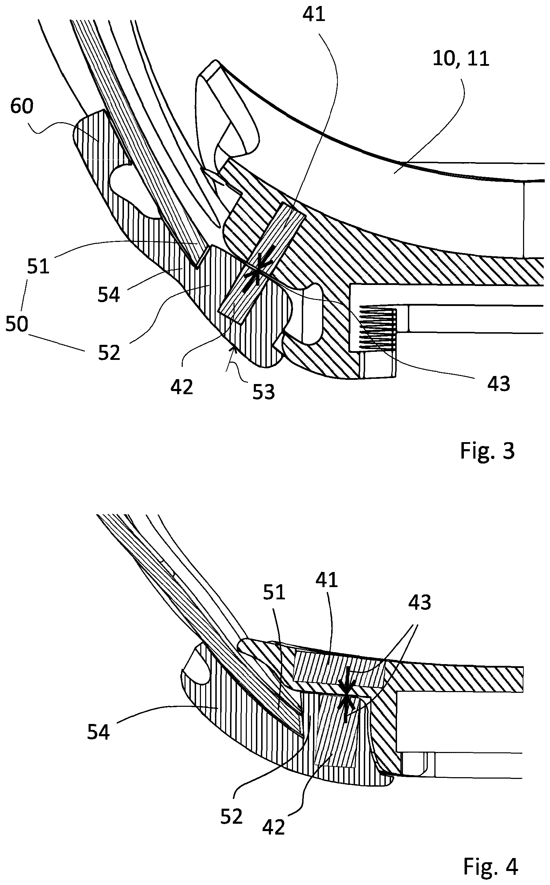

Insertion of a limb, for instance a forearm, into the cuff 1 can be difficult as, in most cases, there is an enlargement at the beginning of the palm. This makes it difficult to insert the arm into the cuff 1 from the proximal side. In order to facilitate the insertion of the arm into the cuff 1 on the proximal side, the design is divided into a cuff base 10 in form of a lower shell 11 and a fixation element 20, which can be a cuff top 30 in form of an upper shell 31, see fig. 1, or a strap system 70, see fig. 3.

As shown in the embodiment of fig. 1, the shells 11, 31 can be separated as two distinct elements. The lower shell 11 can in this case be set radially to the axis of the arm. The upper shell 31 can then be coupled to the lower shell 11 to create a closed system around the forearm.

The number of shells 11, 31 can be larger than two and rise to three, four or more, but that can be the case only if they can be linked in such a way that the user does not need to hold them all in position before tightening them together around their forearm.

The way in which the shells can be connected to each other should be easy and effortless. In the embodiment shown in fig. 1, the upper shell 31 as the fixation element 20 is connected to the lower shell 11 by means of the lacing system 60, which comprises second form fit means 52, which is adapted to allow a geometrical interlocking with first form fit means 51 of the lower shell 11 on demand. Through this, the second form fit means 52 and the first form fit means create a form fit connection 50.

This means that the second form fit means 52 can be disconnected from the lower shell 11 whenever it is desired. The second form fit means 52 acts as a hook.

Each second form fit means 52 is provided at a lacing element 61 in form of a cable, which is connected with a lacing knob 62. By turning of the lacing knob 62, tension is generated in the lacing element, which causes the tightening of the lacing system 60.

As the tension generated in the lacing system 60 increases, the circumference becomes reduced, allowing the lacing system 60 to be tightened around the forearm which results in better transmission of the forces exerted on the cuff 1.

The fixation element 20 in form of the upper shell 31 comprising the lacing system 60 may comprise according to the embodiment shown in fig. 1 a common part 54 at which a plurality of the second form fit means 52 are provided. Thus, at the lower shell 11 a number of the first form fit means 51 is provided accordingly.

As shown in fig. 2 to 4, in order to increase the user friendliness, at least one first magnetic part 41 is positioned in the body of the lower shell 11, and at least one second magnetic part 42 is positioned in the second form fit means 52 at the lacing system. In the case of the use of a common part at which a plurality of the second form fit means 52 are provided, a corresponding number of second magnetic parts 42 is provided at this common part 54, too.

Thus, the second magnetic part 43 and therefore the hook of the second form fit means is guided by the attracting magnetic force 43 of both magnetic parts 41, 42 into the first form fit means 51 in the lower shell 11 along the insertion direction 53, shown in fig. 2.

The magnetic parts 41, 42 then hold the form fit means 51, 52 in place by means of the attracting magnetic force 43, therefore creating a magnetic connection 40, see fig. 3. Once tension is set in the lacing system 61, the magnetic parts 41, 42 no longer play a role, since the form fit means 51, 52 are geometrically interlocked, for instance by means of an undercut, shown in fig. 2.

Once the form fit means 51, 52 are in place in the lower shell 11, the lacing system 60 can start to be operated. The higher the cable tension, the more difficult it becomes to remove the form fit means 51, 52. The cuff top 30 slides on the cuff base 10 when the lacing system 60 gets tightened.

The number of form fit means 51, 52 can vary, and the form fit means 51, 52 can be positioned at different positions, as shown in fig. 4. The interlocking form fit means 51, 52 can also be integrated into the shells, in this case the shells must be deformed when the lacing system 60 is being operated.

In order to remove the cuff 1, the user must release the tension of the lacing system 60 and pull radially on the second form fit means 52, or on the lacing element 61. The cuff 1 can be removed, once the second form fit means 52 is released.

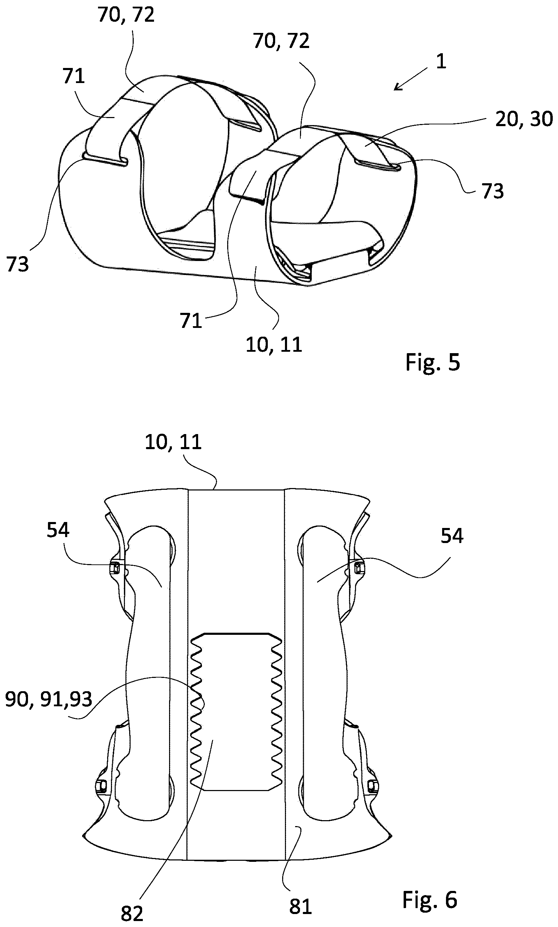

In fig. 5, a different embodiment of the cuff 1 is shown. In this embodiment, the cuff 1 provides a cuff base 10 in form of a lower shell 11 and a fixation means 20 in form of a strap system 70.

The strap system 70 comprises two straps 71 which are led through slits 73 of the cuff base 10. The straps 71 are provided with a hook-and-loop fastener 72, respectively.

By tensioning the straps 71 and fixation of the hook-and-loop fasteners 72, the strap system 70 can be tightened around the forearm.

The embodiment of the cuff 1 comprising a strap system 70 is not restricted to the embodiment shown in fig. 5.

A further embodiment not shown here may comprise a hook-based closure, similar or equal to the common part 54 at which a plurality of the second form fit means 52 and/ or second magnetic parts 42 are provided and which is shown in fig. 1.

When the user places the cuff base 10 on his arm, the second form fit means 52 approaches its position, thanks to the attracting magnetic force 43 of the magnetic parts 41, 42. The user can tighten the strap system 70 with one hand thereby achieving an adjusted tighter or looser fit of the cuff 1 on the arm.

In both embodiments shown in fig. 1 and 3, cushioning material 80 is applied in the cuff base 10.

Another aspect of the design is its modularity.

The system allows for the connection of different modules to best meet the needs of the users. The module 100 can be inserted into the lower shell 11 of the cuff 1.

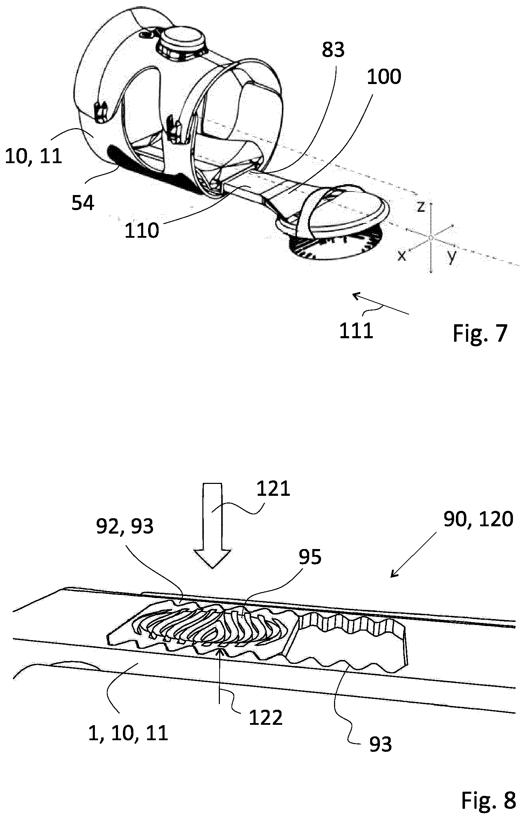

As shown in fig. 7, an insertion element 110 of a module 100 can be directly inserted along a slide-in direction 111 into a retainer 82, which is provided in the cuff base 10 through an insertion opening 83 which in turn is arranged at the distal end of the cuff 1, shown in fig. 6.

Once the insertion element 110 of the module 100 is inserted in the insertion opening 83, the user has to press a button 95, shown in fig. 8 to 11, and slide the insertion element 110 of the module 100 into the lower shell 11, while keeping the button 95 pressed. Here, the button 95 is a special design on the insertion element 110 of the module 100, which slides into the lower shell 11. The user can then release the button 95 when the desired position is reached.

For fixation of different positions of the module 100 with regard to the cuff 1, the orthotic system comprising the cuff 1 and the module 100 further comprises a ratchet system 90. The ratchet system 90 comprises a first part 91 provided at the lower shell 11 of the cuff 1, as well as a second part 92 provided at the insertion element 110 of the module 100 and/ or at the button 95. Both components 91, 92 of the ratchet system 90 feature form elements 93, each having a shape like a tooth. In an embodiment, such tooth may have a rounded shape. The form elements 93 of the first part 91 and the second part 92 are complementary to each other in form and size.

Positive locking 120 of these form elements 93 allows the transfer of loads along the y-axis, shown in fig. 7.

Furthermore, the module 100 can slide along the y-axis in the slide-in direction 111 parallel to an arm axis. Depending on the activity and intensity, different positions of the module 100 along the y-axis might be required. This allows for the same design to be used by users with different arm lengths.

The distance between the area of interest of the module 100 and the insertion opening of the lower shell 11 as seen in fig. 7 can be adjusted by means of the ratchet system 90.

The number of positions of the module 100 is determined by the number of form elements 93 on the first part 91 and the second part 92 of the ratchet system 90.

Fig. 8 to 11 show a method for changing the position of the module with respect to the cuff 1.

In fig. 8 to 11 a part of the lower shell 11 or the cuff base 10 of the cuff 1 is shown, as well as a part of the insertion element 110 of a module, which is not shown completely.

At the insertion element 110 the button 95 is provided.

In the retainer 82 of the lower shell 11 a first part 91 of the ratchet system 90 is provided, comprising a plurality of form elements 93 in the shape of tooth, respectively.

At the insertion element 110 and/ or at the button a second part 92 of the ratchet system 90 is provided, comprising also a plurality of form elements 93 in the shape of a tooth, respectively.

The form elements 93 of the first part 91 and of the second part 92 of the ratchet system 90 are arranged in rows, respectively, and are complementary to each other in form and size.

Fig. 8 shows a state in which the form elements 93 of the first part 91 and the second part 92 engage with each other. Thus, a positive locking 120 is established between the lower shell 11 and the insertion element 110. This engaged state is maintained by means of a force 122 acting on the insertion element 110 due to elasticity of the insertion element 110.

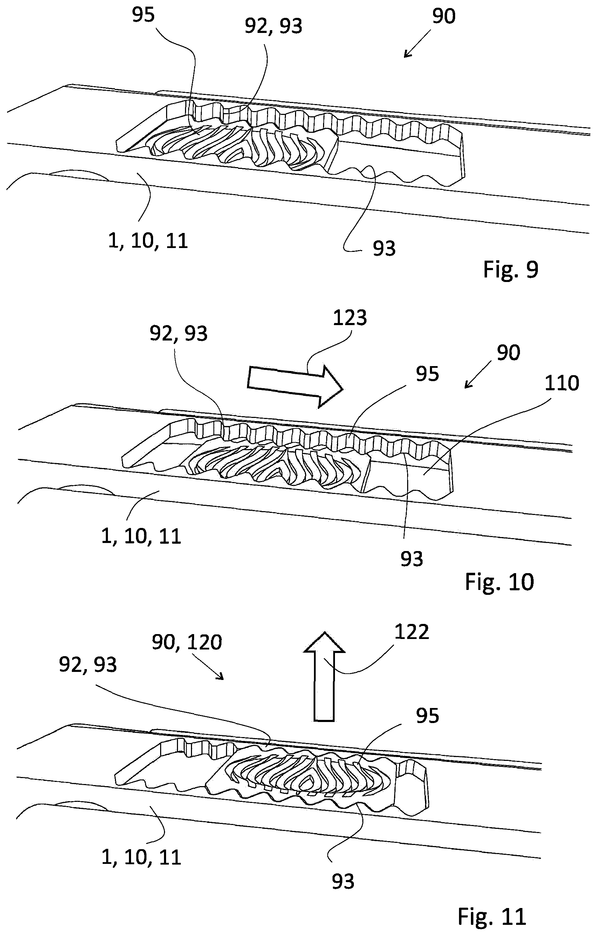

When the button 95 is pressed by a pressing force 121, for instance manually, the button 95 and therefore the insertion element 110 is pressed inward against the elastic force 122, wherein the form elements 93 of the first part 91 and of the second part 92 of the ratchet system 90 are moved out of their engagement, so that the positive locking 120 does not exist anymore, as shown in fig. 9.

When the insertion element 110 has been moved inward, by applying a moving force 123 on the button 95, the insertion element 110 can be moved parallel to the extension of the rows of form elements 93 of the first part 91 and of the second part 92 of the ratchet system 90. Thus, the position of the insertion element 110 and therefore the position of the module with respect to the cuff 1 can be adjusted, see fig. 10.

When the foreseen position has been reached, the button 95 can be released and due to elastic force 122 the insertion element 110 as well as the button moves so that the form elements 93 of the first part 91 and of the second part 92 of the ratchet system 90 engage again, shown in fig. 11.

The module can be removed from the cuff, when necessary, for example, if the targeted activity changes, requiring another module.

The system allows for outdoor activities such as cycling and can also be used in the water, for example, when kayaking. Both of these activities require a critical degree of freedom so as not to restrict the movement of the upper limbs. The system then allows for symmetrical body development through the activities being practised. Furthermore, it can be used for muscle-strengthening sessions or fitness training.

### List of reference signs:

- 1: Cuff
- 10: cuff base
- 11: lower shell
- 20: fixation element
- 30: cuff top
- 31: upper shell
- 40: magnetic connection
- 41: first magnetic part
- 42: second magnetic part
- 43: attracting magnetic force
- 50: form fit connection
- 51: first form fit means
- 52: second form fit means
- 53: insertion direction
- 54: common part
- 60: lacing system
- 61: lacing element
- 62: lacing knob
- 70: strap system
- 71: strap
- 72: hook-and-loop fastener
- 73: slit
- 80: cushioning material
- 81: distal end
- 82: retainer
- 83: insertion opening
- 90: ratchet system
- 91: first part of the ratchet system
- 92: second part of the ratchet system
- 93: form element
- 95: button
- 100: module
- 110: insertion element
- 111: slide-in direction
- 120: positive locking
- 121: pressing force
- 122: force
- 123: moving force

## Claims

1. Cuff (1) combinable with accessories for forming an orthotic system, comprising a cuff base (10) in form of a lower shell (11) and a fixation element (20) which can be combined or is combined with the lower shell (11) into a sleeve attachable to a limb through direct or indirect interlocking with each other, wherein the lower shell (11) and the fixation element (20) provide a system that can be tightened around the limb by reducing a circumference formed by the lower shell (11) and the fixation element (20), such that load transfer from an accessory to the limb is enabled,
wherein the lower shell (11) comprises at least one first form fit means (51) and the fixation element (20) comprises at least one second form fit means (52), wherein both form fit means (51,52) are adapted to realize a form fit connection (50), **characterized in that**, said interlocking between the lower shell and the fixation element comprises a magnetic connection, wherein in a body of the lower shell at least one first magnetic part is provided and in or at the fixation element at least one second magnetic part is provided such that by means of an attracting magnetic force between both magnetic parts the fixation element can be moved onto one side of the body of the lower shell or is or can be held at one side of the body of the lower shell; and **in that** said form fit connection (50) provides an undercut in order to prevent release of the form fit connection (50) when subjected to a force essentially perpendicular to an insertion direction (53) of the form fit connection (50).

2. Cuff according to claim 1, wherein the fixation element (20) is an upper shell (31).

3. Cuff according to claim 2, wherein the cuff (1) provides at least one interlocking member in form of at least one lacing element (61), which is mechanically connected to both shells (11,31) when the interlocking member is interlocked, wherein the lacing element (61) is adapted in such a way that when shortening an effective length of the lacing element (61), a distance between the two shells (11,31) is reduced.

4. Cuff according to claim 3, wherein the lacing element (61) comprises one of the second magnetic parts (42) and/ or one of the second form fit means (52).

5. Cuff according to at least one of the preceding claims, wherein the fixation element (20) is a strap system (70).

6. Cuff according to claim 5, wherein the strap system (70) comprises at least one of the second magnetic parts (42) and/ or one of the second form fit means (52).

7. Cuff according to at least one of the claims 5 and 6, wherein at least one strap (71) of the strap system (70) comprises a hook-and-loop fastener (72).

8. Orthotic system, comprising a cuff (1) according to at least one of the claims 1-7 and a module (100) mechanically connected or connectable to the cuff (1), wherein the cuff (1) comprises a first part (91) of a ratchet system (90) and the module (100) comprises a second part (92) of the ratchet system (90), wherein the first part (91) and the second part (92) have form elements (93), respectively, which are complementary in form and size.

9. Orthotic system according to claim 8, wherein one of the elements cuff (1) and module (100) comprises a retainer (82) provided with one of the first part (91) or second part (92) of the ratchet system and the respective other element of cuff (1) and module (100) comprises an insertion element (110) for positioning in the retainer (82), wherein the insertion element (110) is provided with the respective other part of the first part (91) or second part (92) of the ratchet system and is designed to be pushed into the retainer (82) along a slide-in direction (111), and due to an application of force (122) to bring the first part (91) and second part (92) in a positive locking (120).

10. Orthotic system according to claim 9, wherein the application of force (122) is realized by an elastic behaviour of the insertion element (110) pushing the respective first part (91) or second part (92) of the ratchet system in the direction of the respective first part (91) or second part (92) at the retainer in order to establish the positive locking (120), wherein the Young's modulus of the insertion element is between 0.5 GPa and 7 GPa.

11. Orthotic system according to claim 9, wherein the insertion element (110) is adapted in such a way that the insertion element (110) can be manually pushed and thus moved by a pressing force of at least 3 N so that its first part (91) or second part (92) of the ratchet system disengages from the positive locking (120) with the respective first part (91) or second part (92) provided at the retainer.

12. Orthotic system according to at least one of the claims 9 to 11, wherein a plurality of first parts (91) and second parts (92) are arranged in rows, respectively, so that the positive locking (120) can be realized at different positions along the slide-in direction (111).

## Patentansprüche

1. Manschette (1), kombinierbar mit Zubehörteilen, zum Ausbilden eines Orthesesystems, umfassend eine Manschettenbasis (10) in Form einer unteren Schale (11) und ein Befestigungselement (20), das mit der unteren Schale (11) zu einer Hülle kombiniert werden kann oder kombiniert ist, die durch direkte oder indirekte Verschränkung an einer Gliedmaße angebracht werden kann, wobei die untere Schale (11) und das Befestigungselement (20) ein System bereitstellen, das durch Reduzieren eines von der unteren Schale (11) und dem Befestigungselement (20) ausgebildeten Umfangs um die Gliedmaße herum festgezogen werden kann, sodass eine Verlagerung der Belastung von einem Zubehörteil auf die Gliedmaße ermöglicht wird,
wobei die untere Schale (11) wenigstens ein erstes Formschlussmittel (51) umfasst und das Befestigungselement (20) wenigstens ein zweites Formschlussmittel (52) umfasst, wobei beide Formschlussmittel (51,52) dazu angepasst sind, eine Formschlussverbindung (50) zu realisieren, **dadurch gekennzeichnet, dass** die Verschränkung zwischen der unteren Schale und dem Befestigungselement eine magnetische Verbindung umfasst, wobei in einem Körper der unteren Schale wenigstens ein erstes magnetisches Teil bereitgestellt wird und in oder an dem Befestigungselement wenigstens ein zweites magnetisches Teil bereitgestellt wird, sodass das Befestigungselement mittels einer anziehenden Magnetkraft zwischen beiden magnetischen Teilen auf eine Seite des Körpers der unteren Schale bewegt werden kann oder wird oder an einer Seite des Körpers der unteren Schale gehalten werden kann; und dadurch, dass die Formschlussverbindung (50) einen Unterschnitt bereitstellt, um ein Lösen der Formschlussverbindung (50) zu verhindern, wenn sie einer Kraft unterliegt, die im Wesentlichen senkrecht zu einer Einführrichtung (53) der Formschlussverbindung (50) ist.

2. Manschette nach Anspruch 1, wobei das Befestigungselement (20) eine obere Schale (31) ist.

3. Manschette nach Anspruch 2, wobei die Manschette (1) wenigstens ein Verschränkungselement in Form wenigstens eines Schnürelements (61) bereitstellt, das mechanisch mit beiden Schalen (11,31) verbunden wird, wenn das Verschränkungselement verschränkt wird, wobei das Schnürelement (61) so angepasst ist, dass bei Verkürzung einer effektiven Länge des Schnürelements (61) ein Abstand zwischen den zwei Schalen (11,31) reduziert wird.

4. Manschette nach Anspruch 3, wobei das Schnürelement (61) eines der zweiten magnetischen Teile (42) und/oder eines der zweiten Formschlussmittel (52) umfasst.

5. Manschette nach mindestens einem der vorangehenden Ansprüche, wobei das Befestigungselement (20) ein Riemensystem (70) ist.

6. Manschette nach Anspruch 5, wobei das Riemensystem (70) wenigstens eines der zweiten magnetischen Teile (42) und/oder eines der zweiten Formschlussmittel (52) umfasst.

7. Manschette nach mindestens einem der Ansprüche 5 und 6, wobei wenigstens ein Riemen (71) des Riemensystems (70) einen Klettverschluss (72) umfasst.

8. Orthesesystem, das Folgendes umfasst: eine Manschette (1) nach wenigstens einem der Ansprüche 1-7 und ein Modul (100), das mechanisch mit der Manschette (1) verbunden ist oder verbindbar ist, wobei die Manschette (1) einen ersten Teil (91) eines Ratschensystems (90) umfasst und das Modul (100) einen zweiten Teil (92) des Ratschensystems (90) umfasst, wobei der erste Teil (91) und der zweite Teil (92) jeweils ein Formelement (93) aufweisen, das in Form und Größe komplementär ist.

9. Orthesesystem nach Anspruch 8, wobei eines der Elemente der Manschette (1) und des Moduls (100) eine mit einem des ersten Teils (91) oder des zweiten Teils (92) des Ratschensystems versehene Halterung (82) umfasst und das jeweils andere Element der Manschette (1) und des Moduls (100) ein Einführelement (110) zum Positionieren in der Halterung (82) umfasst, wobei das Einführelement (110) mit dem jeweils anderen Teil des ersten Teils (91) oder des zweiten Teils (92) des Ratschensystems versehen ist und dazu ausgestaltet ist, entlang einer Einschubrichtung (111) in die Halterung (82) gepresst zu werden und den ersten Teil (91) und den zweiten Teil (92) aufgrund einer Kraftausübung (122) in eine positive Arretierung (120) zu bringen.

10. Orthesesystem nach Anspruch 9, wobei die Kraftausübung (122) durch ein elastisches Verhalten des Einführelements (110) realisiert wird, das entsprechend den ersten Teil (91) oder den zweiten Teil (92) des Ratschensystems entsprechend in die Richtung des ersten Teils (91) oder des zweiten Teils (92) an der Halterung presst, um die positive Arretierung (120) zu erzeugen, wobei der E-Modul des Einführelements zwischen 0,5 GPa und 7 GPa liegt.

11. Orthesesystem nach Anspruch 9, wobei das Einführelement (110) so angepasst ist, dass manuell gegen das Einführelement (110) gepresst und es damit durch eine Presskraft von wenigstens 3 N bewegt werden kann, sodass sich der erste Teil (91) oder der zweite Teil (92) des Ratschensystems aus der an der Halterung bereitgestellten positiven Arretierung (120) entsprechend mit dem ersten Teil (91) oder dem zweiten Teil (92) löst.

12. Orthesesystem nach mindestens einem der Ansprüche 9 bis 11, wobei eine entsprechende Mehrzahl erster Teile (91) oder zweiter Teile (92) in Reihen angeordnet sind, sodass die positive Arretierung (120) an verschiedenen Positionen entlang der Einschubrichtung (111) umgesetzt werden kann.

## Revendications

1. Brassard (1) pouvant être combinée à des accessoires pour former un système orthétique, comprenant une base de brassard (10) sous la forme d'une coque inférieure (11) et un élément de fixation (20) qui peut être combiné ou est combiné à la coque inférieure (11) en un manchon pouvant être relié à un membre par verrouillage direct ou indirect l'un avec l'autre, dans laquelle la coque inférieure (11) et l'élément de fixation (20) fournissent un système qui peut être serré autour du membre en réduisant une circonférence formée par la coque inférieure (11) et l'élément de fixation (20) de sorte qu'un transfert de charge d'un accessoire au membre est permis,
dans laquelle la coque inférieure (11) comprend au moins un premier moyen de conjugaison de formes (51) et l'élément de fixation (20) comprend au moins un second moyen de conjugaison de formes (52), dans laquelle les deux moyens de conjugaison de formes (51, 52) sont adaptés à réaliser une connexion par conjugaison de formes (50), **caractérisée en ce que** ledit verrouillage entre la coque inférieure et l'élément de fixation comprend une connexion magnétique, dans laquelle dans un corps de la coque inférieure, au moins une première partie magnétique est prévue et dans ou au niveau de l'élément de fixation, au moins une seconde partie magnétique est prévue de sorte qu'au moyen d'une force magnétique d'attraction entre les deux parties magnétiques, l'élément de fixation peut être déplacé sur un côté du corps de la coque inférieure ou est ou peut être maintenu sur un côté du corps de la coque inférieure ; et **en ce que** ladite connexion par conjugaison de formes (50) fournit une contre-dépouille afin d'empêcher la libération de la connexion par conjugaison de formes (50) lors de la soumission à une force essentiellement perpendiculaire à une direction d'insertion (53) de la connexion par conjugaison de formes (50).

2. Brassard selon la revendication 1, dans laquelle l'élément de fixation (20) est une coque supérieure (31).

3. Brassard selon la revendication 2, dans laquelle le brassard (1) fournit au moins un élément de verrouillage sous la forme d'au moins un élément de laçage (61) qui est connecté mécaniquement aux deux coques (11, 31) lorsque l'élément de verrouillage est verrouillé, dans laquelle l'élément de laçage (61) est adapté de telle sorte que lors du raccourcissement d'une longueur réelle de l'élément de laçage (61), une distance entre les deux coques (11, 31) est réduite.

4. Brassard selon la revendication 3, dans laquelle l'élément de laçage (61) comprend une des secondes parties magnétiques (42) et/ou un du second moyen de conjugaison de formes (52).

5. Brassard selon au moins une des revendications précédentes, dans laquelle l'élément de fixation (20) est un système de sangles (70).

6. Brassard selon la revendication 5, dans laquelle le système de sangles (70) comprend au moins une des secondes parties magnétiques (42) et/ou un du second moyen de conjugaison de formes (52).

7. Brassard selon au moins une des revendications 5 et 6, dans laquelle au moins une sangle (71) du système de sangles (70) comprend un système de fermeture autoagrippante (72).

8. Système orthétique, comprenant un brassard (1) selon au moins une des revendications 1 à 7 et un module (100) connecté ou pouvant être connecté mécaniquement au brassard (1), dans lequel le brassard (1) comprend une première partie (91) d'un système de cliquet (90) et le module (100) comprend une seconde partie (92) du système de cliquet (90), dans lequel la première partie (91) et la seconde partie (92) ont des éléments de forme (93), respectivement, qui sont complémentaires en forme et taille.

9. Système orthétique selon la revendication 8, dans lequel un des éléments que sont le brassard (1) et le module (100) comprend un dispositif de retenue (82) pourvu d'une de la première partie (91) ou seconde partie (92) du système de cliquet et l'autre élément respectif du brassard (1) et du module (100) comprend un élément d'insertion (110) pour le positionnement dans le dispositif de retenue (82), dans lequel l'élément d'insertion (110) est pourvu de l'autre partie respective de la première partie (91) ou seconde partie (92) du système de cliquet et est conçu pour être poussé dans le dispositif de retenue (82) le long d'une direction de coulissement (111), et en raison d'une application de force (122) pour amener les première partie (91) et seconde partie (92) dans un verrouillage positif (120).

10. Système orthétique selon la revendication 9, dans lequel l'application de force (122) est réalisée par un comportement élastique de l'élément d'insertion (110) poussant la première partie (91) ou seconde partie (92) respective du système de cliquet dans la direction de la première partie (91) ou seconde partie (92) respective au niveau du dispositif de retenue afin d'établir le verrouillage positif (120), dans lequel le module de Young de l'élément d'insertion est compris entre 0,5 GPa et 7 GPa.

11. Système orthétique selon la revendication 9, dans lequel l'élément d'insertion (110) est adapté de telle sorte que l'élément d'insertion (110) peut être poussé manuellement et ainsi déplacé par une force de pression d'au moins 3 N de sorte que sa première partie (91) ou seconde partie (92) du système de cliquet se désengage du verrouillage positif (120) avec la première partie (91) ou seconde partie (92) respective prévue au niveau du dispositif de retenue.

12. Système orthétique selon au moins une des revendications 9 à 11, dans lequel une pluralité de premières parties (91) et secondes parties (92) sont agencées en rangées, respectivement, de sorte que le verrouillage positif (120) peut être réalisé à différentes positions le long de la direction de coulissement (111).
